Europäisches Patentamt
European Patent Office
Office européen des brevets

(19) (11) Veröffentlichungsnummer: **0 173 205 A2**

# (12) EUROPÄISCHE PATENTANMELDUNG

(21) Anmeldenummer: **85110354.9**

(22) Anmeldetag: **19.08.85**

(51) Int. Cl.⁴: **C 07 D 237/04**, C 07 D 491/048, A 61 K 31/50, A 61 K 31/495 // (C07D491/048, 307:00, 237:00)

(30) Priorität: **29.08.84 DE 3431700**

(43) Veröffentlichungstag der Anmeldung: **05.03.86 Patentblatt 86/10**

(84) Benannte Vertragsstaaten: **AT BE CH DE FR GB IT LI SE**

(71) Anmelder: **BAYER AG, Konzernverwaltung RP Patentabteilung, D-5090 Leverkusen 1 Bayerwerk (DE)**

(72) Erfinder: **Franckowiak, Gerhard, Dr., Henselweg 10, D-5600 Wuppertal 1 (DE)**
Erfinder: **Wehinger, Egbert, Dr., Gellertweg 33, D-5600 Wuppertal 1 (DE)**
Erfinder: **Thomas, Günther, Dr., Henselweg 5, D-5600 Wuppertal 1 (DE)**
Erfinder: **Schramm, Matthias, Dr., Paffrather Strasse 38, D-5000 Koeln 80 (DE)**
Erfinder: **Gross, Rainer, Dr., Platzhoffstrasse 23, D-5600 Wuppertal 1 (DE)**

(54) **Neue 1,4-dihydro-pyridazin-derivate, Verfahren zu ihrer Herstellung und ihre Verwendung in Arzneimitteln.**

(57) 1,4-Dihydro-pyridazin-Derivate der allgemeinen Formel (I)

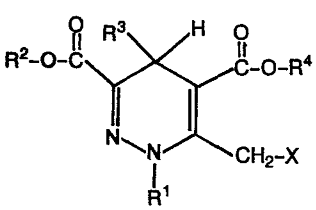

(I)

mit in der Beschreibung näher bezeichneten Substituentendefinitionen für $R_1$, $R_2$, $R_3$ und $R_4$ und X sowie ein Verfahren zu ihrer Herstellung durch Umsetzung von Dihydropyridazinen der Formel II

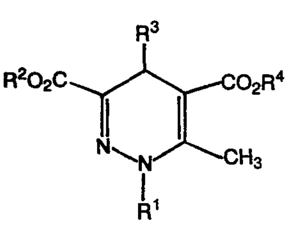

(II)

mit Halogenierungsmitteln in Gegenwart von inerten organischen Lösungsmitteln. Gegebenenfalls kann anschließend cyclisiert werden. Die erfindungsgemäßen Verbindungen können als Arzneimittel verwendet werden.

BAYER AKTIENGESELLSCHAFT 5090 Leverkusen, Bayerwerk
Konzernverwaltung RP
Patentabteilung E/Kü-c

# Neue 1,4-Dihydro-pyridazin-derivate, Verfahren zu ihrer Herstellung und ihre Verwendung in Arzneimitteln

Die vorliegende Erfindung betrifft neue 1,4-Dihydro-6-halogenmethyl-pyridazine und neue 5-Oxo-1,4,5,7-tetrahydro-furo[3,4-c]pyridazin-3-carbonsäureester, Verfahren zu ihrer Herstellung sowie ihre Verwendung in Arzneimitteln, insbesondere in kreislaufbeeinflussenden Arzneimitteln.

Die Erfindung betrifft neue 1,4-Dihydro-pyridazin-Derivate der allgemeinen Formel (I)

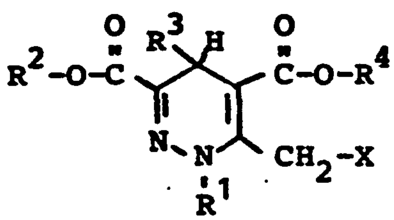

(I)

in welcher

$R^1$ für Wasserstoff,
für einen geradkettigen oder verzweigten Alkylrest,

bevorzugt mit bis zu 8 C-Atomen, besonders bevorzugt mit bis zu 5 C-Atomen, der gegebenenfalls durch 1-2, bevorzugt durch 1 Sauerstoffatom in der Kette unterbrochen ist,

oder für einen Aryl- oder Aralkylrest, bevorzugt mit 6-12 C-Atomen, besonders bevorzugt Phenyl und Benzyl steht,

$R^2$ und $R^4$ gleich oder verschieden sind und jeweils für Wasserstoff, für einen geradkettigen, verzweigten oder cyclischen, gesättigten oder ungesättigten Kohlenwasserstoffrest, bevorzugt mit bis zu 8, besonders bevorzugt mit bis zu 6 C-Atomen, der gegebenenfalls durch 1-2 Sauerstoff- und/oder Schwefelatome und/oder $SO_2$-Gruppen unterbrochen ist und/oder der substituiert sein kann durch Hydroxy, Halogen (bevorzugt F, Cl, Br, besonders bevorzugt mit 1 oder mehreren Fluoratomen), Heteroaryl (bevorzugt 5-7 gliedrig mit den Atomen O,N,S, besonders bevorzugt mit 1-2 Stickstoff-, Sauerstoff- oder Schwefelatomen), eine gegebenenfalls durch Halogen (bevorzugt F, Cl, Br), Cyan, Amino, Trifluormethyl, Alkyl, Alkylamino, Alkoxy (jeweils bevorzugt mit 1-4 C-Atomen) oder Nitro substituierte Phenyl- oder Phenoxygruppe oder durch eine Aminogruppe, wobei diese Aminogruppe gegebenenfalls entweder Wasserstoff und einen Substituenten oder zwei gleiche oder verschiedene Substituenten aus den Gruppen Alkyl, Alkoxyalkyl (bevorzugt jeweils mit 1-4 C-Atomen), Aryl und Aralkyl (bevorzugt mit jeweils

6-12 C-Atomen) trägt und wobei diese Substituenten gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der mit Alkyl (bevorzugt mit 1-4, besonders bevorzugt mit 1-2 C-Atomen) substituiert sein kann,

für Aryl, bevorzugt mit 6-10 C-Atomen, besonders bevorzugt für Phenyl, der gegebenenfalls 1-2 gleiche oder verschiedene Substituenten tragen kann, wobei als Substituenten geradkettiges oder verzweigtes Alkyl (bevorzugt mit bis zu 4 C-Atomen, besonders bevorzugt mit bis zu 2 C-Atomen), Halogen (bevorzugt F, Cl, Br), Nitro, Trifluormethyl oder Trifluormethoxy gelten,

oder für einen Heteroaromaten bevorzugt Pyridyl, Furyl, Thienyl, stehen,

$R^3$ für einen Arylrest, bevorzugt mit 6-10, besonders bevorzugt mit 6 C-Atomen,

oder für Heteroaryl, bevorzugt für Pyrryl, Furyl, Thienyl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzoxadiazolyl, Benzimidazolyl, Chinazolyl oder Chinoxalyl steht,

wobei sowohl der Aryl- als auch der Heteroarylrest gegebenenfalls 1-3, bevorzugt 1-2, gleiche

oder verschiedene Substituenten aus der Reihe Phenyl, Alkyl (mit bevorzugt 1-4, besonders bevorzugt 1-2 C-Atome), Alkoxy (mit bevorzugt 1-4, besonders bevorzugt 1-2 C-Atomen), Halogen (bevorzugt F, Cl, Br), Dioxyalkylen (mit 1-2 C-Atomen), Trifluormethyl, Trifluormethoxy, Cyan, Hydroxy, Azido, Nitro, Carboxy, Alkoxycarbonyl (bevorzugt mit 2-4 besonders bevorzugt mit 2-3 C-Atomen), Carbonamido, Sulfonamido, $SO_2$-$CF_3$, $SO_2$-Alkyl (bevorzugt mit 1-4 besonders bevorzugt mit 1-2 C-Atomen) trägt,

X für Halogen, bevorzugt Brom oder Chlor, steht

oder

X und $R^4$ gemeinsam für eine direkte Bindung stehen,

in Form von Isomeren, Isomerengemischen, Racematen und optischen Antipoden sowie deren physiologisch unbedenklichen Salze.

Bevorzugt sind solche Verbindungen der Formel (I), in welcher

$R^1$ für Wasserstoff,
für gerades oder verzweigtes Alkyl mit bis zu 8 C-Atomen, wobei der Alkylrest gegebenenfalls durch 1-2, bevorzugt 1 Sauerstoffatom in der Kette unterbrochen ist, oder für Aryl, Aralkyl mit jeweils 6-12 C-Atomen steht,

$R^2$ und $R^4$ gleich oder verschieden sind und jeweils

für Wasserstoff,

für einen geradkettigen, verzweigten oder cyclischen, gesättigten oder ungesättigten Kohlenwasserstoffrest mit bis zu 8 C-Atomen, der gegebenenfalls durch 1-2 Sauerstoff- und/oder Schwefelatome und/oder $SO_2$-Gruppen unterbrochen ist und/oder der substituiert sein kann durch Hydroxy, Fluor, Chlor, Brom, Heteroaryl bevorzugt Pyridyl, Thienyl, Furyl, eine gegebenenfalls durch Fluor, Chlor, Brom, Cyan, Trifluormethyl, Alkyl mit 1-4 C-Atomen, Alkoxy mit 1-4 C-Atomen oder Nitro substituierte Phenyl- oder Phenoxygruppe oder durch eine Aminogruppe, wobei diese Aminogruppe gegebenenfalls entweder Wasserstoff und einen oder 2 gleiche oder verschiedene Substituenten aus den Gruppen Alkyl, Alkoxyalkyl (jeweils mit bis zu 4 C-Atomen), Aryl und Aralkyl (mit bis zu 12 C-Atomen) trägt und wobei diese Substituenten gegebenenfalls mit dem Stickstoffatom einen 5-6 gliedrigen Ring bilden, der als weiteres Atom ein Sauerstoff-, Schwefel- oder Stickstoffatom enthalten kann und gegebenenfalls mit Alkyl mit 1-4 C-Atomen substituiert sein kann,

für einen Phenylrest, der gegebenenfalls 1-2 gleiche oder verschiedene Substituenten tragen kann, wobei die Substituenten geradkettiges oder verzweigtes Alkyl mit bis zu 4 C-Atomen, Fluor, Chlor, Brom, Nitro, Trifluormethyl oder Trifluormethoxy sein können,

oder für Pyridyl, Thienyl oder Furyl stehen,

$R^3$ für Phenyl, Naphthyl, für Pyrryl, Furyl, Thienyl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzoxadiazolyl, Benzimidazolyl, Chinazolyl oder Chinoxalyl steht, wobei sowohl Aryl als auch Heteroaryl gegebenenfalls 1-2 gleiche oder verschiedene Substituenten aus der Reihe Phenyl, Alkyl, Alkoxy jeweils mit 1-4 C-Atormen, Fluor, Chlor, Brom, Dioxymethylen, Trifluormethyl,Trifluormethoxy, Cyan, Hydroxy, Azido, Nitro, Carboxy, Alkoxycarbonyl mit 2-4 C-Atomen, Carbonamido, Sulfonamido, $SO_2$-$CF_3$, $SO_2$-Alkyl mit 1-4 C-Atomen tragen,

X für Halogen, bevorzugt Chlor oder Brom, steht

oder

X und $R^4$ gemeinsam eine direkte Bindung darstellen.

Besonders bevorzugt sind Verbindungen der allgemeinen Formel (I),

in welcher

$R^1$ für Wasserstoff, für Alkyl mit bis zu 5 C-Atomen, gegebenenfalls durch ein Sauerstoffatom in der Kette unterbrochen, oder für Phenyl oder Benzyl steht,

$R^2$ und $R^4$ gleich oder verschieden sind und jeweils für Wasserstoff,

für einen geradkettigen, verzweigten oder cyclischen, gesättigten oder ungesättigten Kohlenwasserstoffrest mit bis zu 6 C-Atomen, der gegebenenfalls durch bis zu 2 Sauerstoff- und/oder Schwefelatome und/oder $SO_2$-Gruppen unterbrochen ist und/oder der substituiert sein kann durch Hydroxy, ein oder mehrere Fluor, Pyridyl, Furyl, Thienyl, eine gegebenenfalls durch Fluor, Chlor, Brom, Nitro oder Trifluormethyl substituierte Phenyl- oder Phenoxygruppe oder durch eine Aminogruppe, wobei diese Aminogruppe gegebenenfalls entweder Wasserstoff und einen oder zwei gleiche oder verschiedene Substituenten aus den Gruppen Alkyl, Alkoxy mit jeweils bis zu 2 C-Atomen, Phenyl, Benzyl trägt,

oder wobei diese Substituenten mit dem Stickstoffatom die gegebenenfalls mit Methyl oder Ethyl substituierten Ringsysteme Pyrrolidin, Pyrazolidin, Piperidin, Piperazin, Morpholin, Thiomorpholin bildet,

für einen Phenylrest, der gegebenenfalls 1-2 gleiche oder verschiedene Substituenten tragen kann, wobei die Substituenten Alkyl mit 1-2 C-Atomen, Fluor, Chlor, Brom, Nitro, Trifluormethyl oder Trifluormethoxy sein können,

oder für Pyridyl, Furyl oder Thienyl stehen,

$R^3$ für Phenyl, gegebenenfalls substituiert durch ein oder zwei gleiche oder verschiedene Substituenten aus den Gruppen Alkyl oder Alkoxy, jeweils mit 1-2 C-Atomen, Fluor, Chlor, Brom, Trifluormethyl, Trifluormethoxy, Nitro, Azido und Cyano, oder für Benzoxadiazolyl, Pyridyl, Thienyl oder Furyl steht,

X für Chlor oder Brom steht,

oder

$R^4$ und X gemeinsam eine direkte Bindung darstellen.

Physiologisch unbedenkliche Salze können Salze der erfinfindungsgemäßen Stoffe mit anorganischen und organischen Säuren sein. Es seien beispielhaft genannt: Hydrochloride, Hydrogensulfate, Sulfate, Hydrogenphosphate, Acetate, Maleate, Benzoate, Citrate, Tartrate, Lactate.

Die erfindungsgemäßen Verbindungen stellen eine neuartige Stoffklasse zur Kreislaufbehandlung, zur Beeinflussung des Blutzuckers und des Salz- und Flüssigkeitshaushalts dar und sind somit als eine Bereicherung der Pharmazie anzusehen.

Die erfindungsgemäßen Verbindungen der allgemeinen Formel (Ia), in welcher

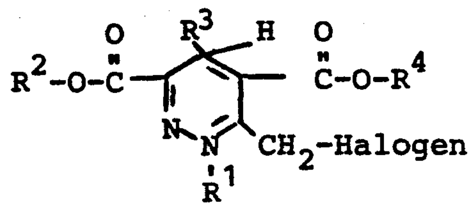

(Ia)

$R^1$ - $R^4$ die oben angegebene Bedeutung haben und

X für Halogen, bevorzugt für Chlor oder Brom steht,

erhält man, wenn man 1,4-Dihydropyridazine der allgemeinen Formel (II)

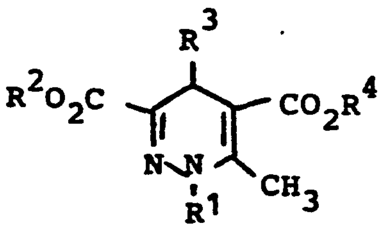

(II)

in welcher

$R^1$ - $R^4$ die oben angegebene Bedeutung haben

mit Halogenierungsmitteln in Gegenwart von inerten organischen Lösungsmitteln, gegebenenfalls in Anwesenheit von Radikalbildnern umsetzt.

Verwendet man als Reaktanden 1,4-Dihydro-6-methyl-4-(2-nitrophenyl)-pyridazin-3,5-dicarbonsäuredimethylester, N-Bromsuccinimid (NBS) mit Azobisisobuttersäurenitril, so kann man den Reaktionsverlauf durch das folgende Formelschema darstellen:

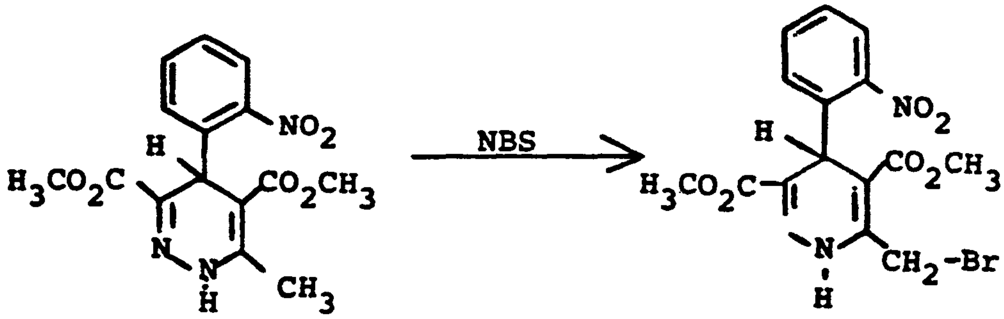

Die als Ausgangsstoffe verwendeten 1,4-Dihydro-6-methyl-3,5-dicarbonsäureester können nach literaturbekannten Methoden dargestellt werden (vgl. EP 9650, US 280 998, DOS 32 39 789).

Als Lösungsmittel kommen alle inerten organischen Lösungsmittel in Frage, bevorzugt jedoch halogenierte Kohlenwasserstoffe wie Dichlor-, Trichlor- oder Tetrachlormethan.

Als Halogenierungsmittel können die üblichen Halogenierungsmittel verwendet werden. Bevorzugt sind Chlor, Brom, N-Chlorsuccinimid oder N-Bromsuccinimid, gegebenenfalls in Anwesenheit von Radikalbildnern wie Azobisisobuttersäurenitril, Benzoylperoxid oder Licht.

Die Reaktionstemperaturen können in einem größeren Bereich variiert werden. Im allgemeinen arbeitet man zwischen 30°C und 120°C, bevorzugt bei der Siedetemperatur des verwendeten Lösungsmittels.

Die Umsetzung kann bei normalem Druck, aber auch bei erhöhtem Druck durchgeführt werden. Im allgemeinen arbeitet man bei Normaldruck.

Das Mengenverhältnis der Reaktanden zueinander ist beliebig, bevorzugt werden jedoch äquimolare Mengen eingesetzt.

Die erfindungsgemäßen Verbindungen der allgemeinen Formel (Ib) in welcher

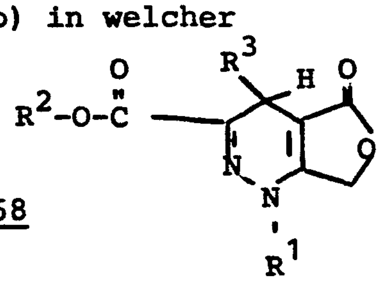

(Ib)

$R^1$ - $R^3$ die oben angegebene Bedeutung haben

und

$R^4$ und X gemeinsam eine direkte Bindung bedeuten,

können hergestellt werden, indem man die 1,4-Dihydropyridazine der Formel (Ia) in welcher

$R^1$ - $R^4$ die oben angegebene Bedeutung haben,

X für Halogen, bevorzugt Chlor oder Brom steht,

mit oder ohne Lösungsmittel pyrolysiert.

Verwendet man als Ausgangsstoff 6-Brommethyl-1,4-dihydro-4-(2-trifluormethylphenyl)-pyridazin-3,5-dicarbonsäuredimethylester, so kann die Reaktion durch folgendes Schema verdeutlicht werden:

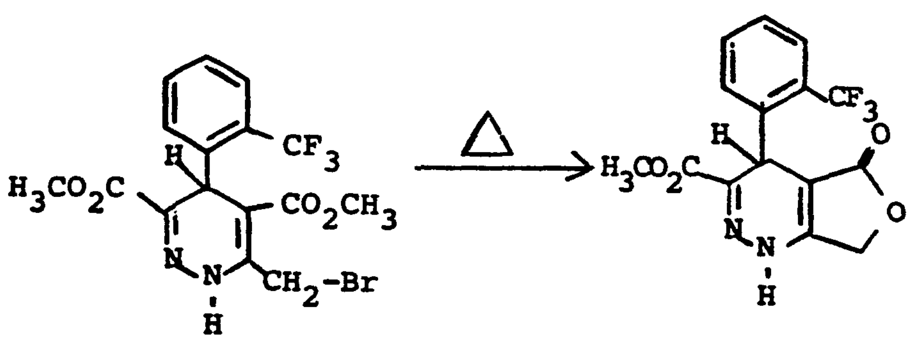

Die Pyrolyse kann mit oder ohne Lösungsmittel durchgeführt werden. Als Lösungsmittel kommen gegebenenfalls alle üblichen inerten organischen Lösungsmittel in Frage. Dazu zählen aromatische Kohlenwasserstoffe wie Benzol, Toluol oder Xylol, Tetralin, Erdölfraktionen, Ether wie Diethylether, Dioxan, Tetrahydrofuran, Glykolmono- bzw. diethylether, Diethylenglykol, Halogenkohlenwasserstoffe wie Di-, Tri- oder Tetrachlormethan, Dichlor- oder Trichlorethylen.

Die Cyclisierung wird in einem Temperaturbereich von 20°C bis 300°C, bevorzugt von 40°C bis 250°C durchgeführt.

Die Pyrolyse kann bei normalem, erhöhtem oder erniedrigten Druck durchgeführt werden. Im allgemeinen arbeitet man bei Normaldruck.

Die erfindungsgemäßen Verbindungen der Formel (I) lassen sich gegebenenfalls nach allgemein bekannten Methoden der Verseifung, Veresterung, Umesterung oder Amidierung zu weiteren erfindungsgemäßen Verbindungen derivatisieren (vgl. Organikum, VEB Deutscher Verlag der Wissenschaftler, Berlin 1967).

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die neuen Verbindungen zeigen ein nicht vorhersehbares und wertvolles pharmakologisches Wirkungsspektrum. Sie können als kreislaufbeeinflussende Mittel zur Beeinflussung des Blutdruckes sowie der Herzkontraktibilität, als Coronartherapeutika oder als Antiarrhythmika dienen. Darüber hinaus können sie zur Beeinflussung des Blutzuckerspiegels und des Salz- und Flüssigkeitshaushaltes eingesetzt werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester , Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und

Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesseren oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung

erfolgt. So kann es in einigen Fällen ausreichend sein, mit weniger als der vorgenannten Mindestmenge auszukommen, während in anderen Fällen die genante obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1

### 6-Brommethyl-1,4-dihydro-4-(2-trifluormethylphenyl)-pyridazin-3,5-dicarbonsäuredimethylester

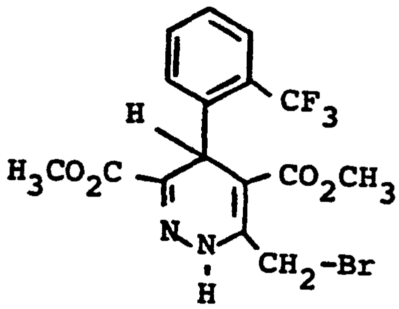

3,56 g (10 mMol) 1,4-Dihydro-6-methyl-4-(2-trifluormethylphenyl)-pyridazin-3,5-dicarbonsäuredimethylester werden in 50 ml Tetrachlorkohlenstoff mit 1,96 g (11 mMol) N-Bromsuccinimid (NBS) unter Zusatz von 100 mg Azobisisobutyronitril 4 Stunden zum Rückfluß erhitzt. Nach Abfiltrieren des Succinimids wird die Lösung eingedampft und der Rückstand aus wenig Isopropanol kristallisiert.

Ausbeute: (72 % der Theorie).
Schmelzpunkt: 217°C (Zers.).

Beispiel 2

6-Brommethyl-1,4-dihydro-4-(3-nitrophenyl)-pyridazin-3,5-dicarbonsäurediethylester

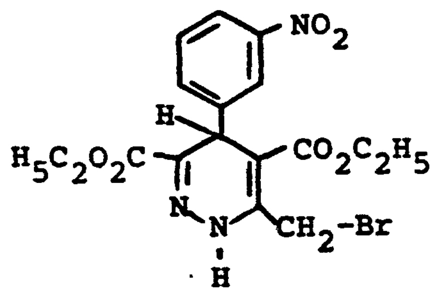

3,61 g (10 mMol) 1,4-Dihydro-6-methyl-4-(3-nitrophenyl)-pyridazin-3,5-dicarbonsäurediethylester werden in 50 ml Tetrachlorkohlenstoff mit 1,96 g (11 mMol) N-Bromsuccinimid unter Zusatz von 100 mg Azobisisobutyronitril analog Beispiel 1 umgesetzt.

Ausbeute: (66 % der Theorie).
Schmelzpunkt: 206°C (Zers.).

Beispiel 3

6-Brommethyl-4-(2-chlorphenyl)-1,4-dihydropyridazin-3,5-dicarbonsäuredimethylester

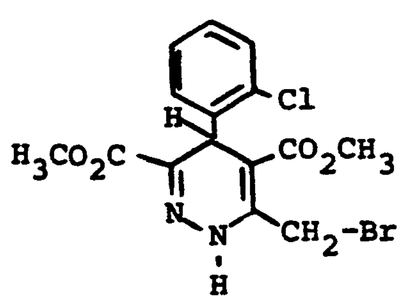

Analog Beispiel 1 werden 3,22 g (10 mMol) 4-(2-Chlorphenyl)-1,4-dihydro-6-methyl-pyridazin-3,5-dicarbonsäuredimethylester mit 1,1 Äquivalenten NBS bromiert.

Ausbeute: (82 % der Theorie)
Schmelzpunkt: 182°C (Zers.).

Beispiel 4

6-Brommethyl-1,4-dihydro-4-(3-nitrophenyl)-pyridazin-3,5-dicarbonsäure-3-(2-methoxyethyl)-5-ethylester

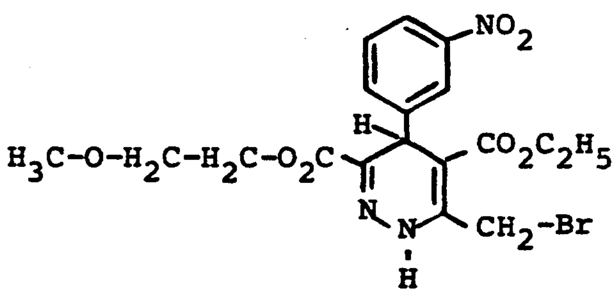

Analog Beispiel 1 werden 1,86 g (5 mMol) 1,4-Dihydro-6-methyl-4-(3-nitrophenyl)-pyridazin-3,5-dicarbonsäure-3-(2-methoxyethyl)-5-ethylester mit 1,1 Äquivalenten NBS bromiert.

Ausbeute: (55 % der Theorie).
Schmelzpunkt: 143°C.

Beispiel 5

6-Brommethyl-1,4-dihydro-4-(3-nitrophenyl)-pyridazin-3,5-dicarbonsäure-3-methyl-5-isopropylester

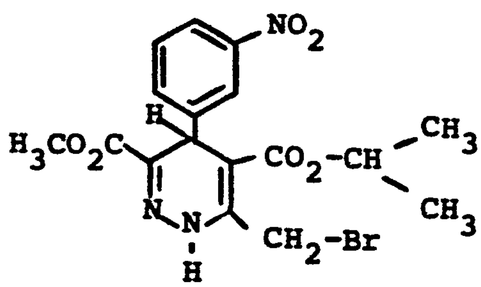

Analog Beispiel 1 werden 1,81 g (5 mMol) 1,4-Dihydro-6-methyl-4-(3-nitrophenyl)-pyridazin-3,5-dicarbonsäure-3-methyl-5-isopropylester mit 1,1 Äquivalenten NBS umgesetzt.

Ausbeute: (52 % der Theorie).
Schmelzpunkt: 105°C (Zers.).

Beispiel 6

6-Brommethyl-4-(2-chlorphenyl)-1,4-dihydro-pyridazin-3,5-dicarbonsäure-3-cyclopentyl-5-methylester

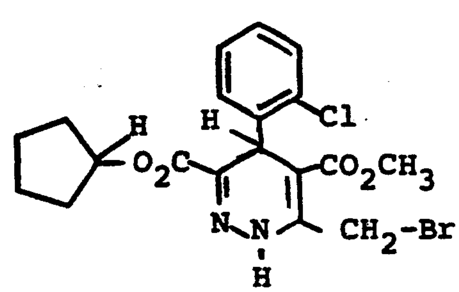

Analog Beispiel 1 werden 1,88 g (5 mMol) 4-(2-Chlorphenyl)-1,4-dihydro-6-methyl-pyridazin-3,5-dicarbonsäure-3-cyclopentyl-5-methylester mit NBS umgesetzt.

Ausbeute: (69 % der Theorie).
Schmelzpunkt: 134°C (Zers.).

Beispiel 7

6-Brommethyl-4-(2-cyanophenyl)-1,4-dihydro-pyridazin-3,5-dicarbonsäure-3-methyl-5-ethylester

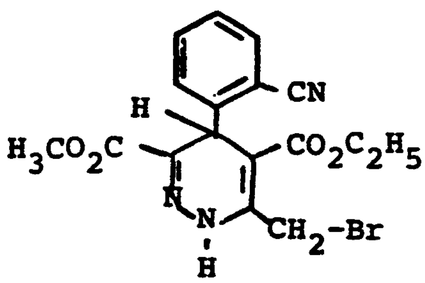

Analog Beispiel 1 werden 1,63 g (5 mMol) 4-(2-Cyanophenyl)-1,4-dihydro-6-methylpyridazin-3,5-dicarbonsäure-3-methyl-5-ethylester mit NBS bromiert.

Ausbeute: (73 % der Theorie).
Schmelzpunkt: 206°C (Zers.).

Beispiel 8

6-Brommethyl-4-(2-chlor-5-nitrophenyl)-1,4-dihydro-pyridazin-3,5-dicarbonsäuredimethylester

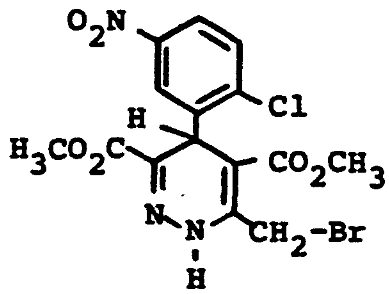

4-(2-Chlor-5-nitrophenyl)-1,4-dihydro-6-methyl-pyridazin-3,5-dicarbonsäuredimethylester wird analog Beispiel 1 mit 1,1 Äquivalent NBS bromiert.

Ausbeute: (71 % der Theorie).
Schmelzpunkt: 216°C.

Beispiel 9

5-Oxo-4-(2-trifluormethylphenyl)-1,4,5,7-tetrahydro-furo[3,4-c]pyridazin-3-carbonsäuremethylester

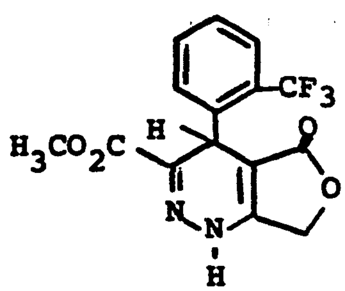

1,3 g (3 mMol) 6-Brommethyl-1,4-dihydro-4-(2-trifluormethylphenyl)-pyridazin-3,5-dicarbonsäuredimethylester werden für 3 Min. zur Schmelze erhitzt. Der Rückstand wird aus Isopropanol umkristallisiert.

Ausbeute: (69 % der Theorie).
Schmelzpunkt: 187°C.

Beispiel 10

4-(3-Nitrophenyl)-5-oxo-1,4,5,7-tetrahydro-furo/3,4-c/pyridazin-3-carbonsäureethylester

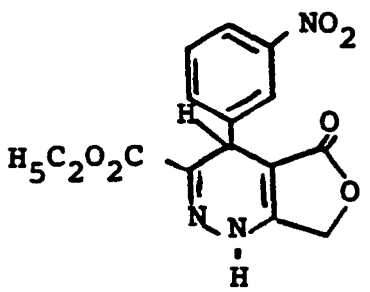

Analog Beispiel 9 wird 6-Brommethyl-1,4-dihydro-4-(3-nitrophenyl)-pyridazin-3,5-dicarbonsäurediethylester pyrolysiert.

Ausbeute: (39 % der Theorie).
Schmelzpunkt: 219°C.

Beispiel 11

4-(2-Chlorphenyl)-5-oxo-1,4,5,7-tetrahydro-furo/3,4-c/pyridazin-3-carbonsäuremethylester

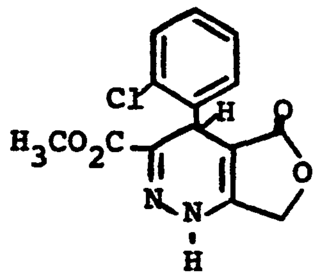

Analog Beispiel 9 wird das Produkt aus Beispiel 3 pyrolysiert.

Ausbeute: (69 % der Theorie).
Schmelzpunkt: 217°C.

Beispiel 12

4-(3-Nitrophenyl)-5-oxo-1,4,5,7-tetrahydro-furo/3,4-c/pyridazin-3-carbonsäure-(2-methoxyethyl)-ester

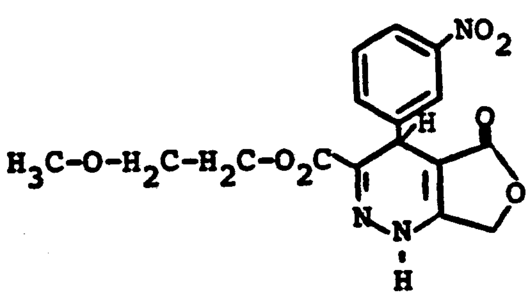

Analog Beispiel 9 wird das Produkt aus Beispiel 4 pyrolysiert.

Ausbeute: (50 % der Theorie).
Harz.

Beispiel 13

4-(3-Nitrophenyl)-5-oxo-1,4,5,7-tetrahydro-furo/3,4-c/pyridazin-3-carbonsäuremethylester

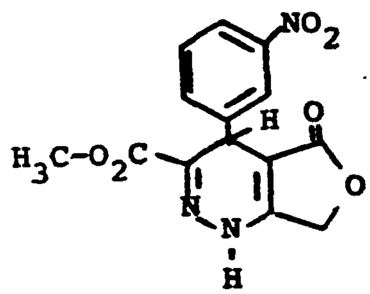

Das Produkt aus Beispiel 5 wird in Diethylenglykol für 2 h auf 120-150° erhitzt. Anschließend wird mit Wasser verdünnt und das Produkt mit Methylenchlorid extrahiert. Der Eindampfrückstand kristallisiert aus Methanol.

Ausbeute: (51 % der Theorie).
Schmelzpunkt: 219°C.

Beispiel 14

4-(2-Chlorphenyl)-5-oxo-1,4,5,7-tetrahydro-furo[3,4-c]pyridazin-3-carbonsäurecyclopentylester

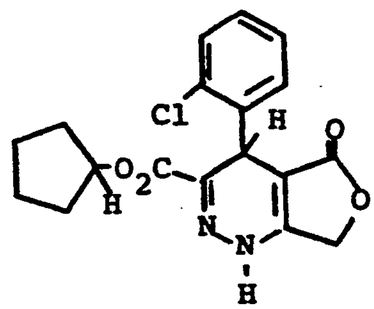

Die Brommethylverbindung aus Beispiel 6 wird, wie unter Beispiel 13 beschrieben, pyrolysiert.

Ausbeute: (49 % der Theorie).

Beispiel 15

4-(2-Cyanophenyl)-5-oxo-1,4,5,7-tetrahydro-furo[3,4-c]pyridazin-3-carbonsäuremethylester

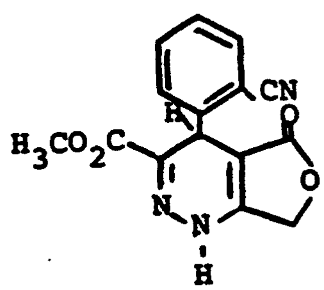

Analog Beispiel 9 wird das Brommethylprodukt aus Beispiel 7 durch Erhitzen auf 190° pyrolysiert.

Ausbeute: (51 % der Theorie).
Schmelzpunkt: 164°C.

Beispiel 16

4-(2-Chlor-5-nitrophenyl)-5-oxo-1,4,5,7-tetrahydro-furo
/3,4-c/pyridazin-3-carbonsäuremethylester

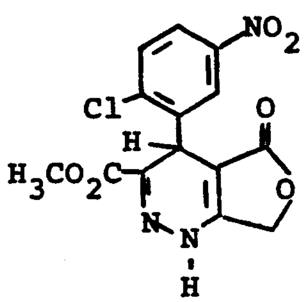

Analog Beispiel 9 wird die Brommethylverbindung aus Beispiel 8 am Schmelzpunkt pyrolysiert.

Ausbeute: (65 % der Theorie).
Schmelzpunkt: 243°C.

Beispiel 17

5-Oxo-4-(3-pyridyl)-1,4,5,7-tetrahydro-furo[3,4-c]pyridazin-3-carbonsäuremethylester

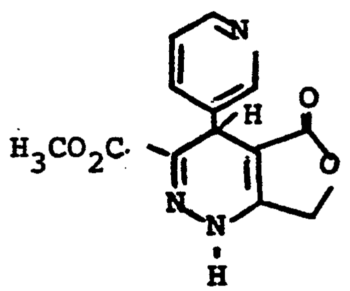

3,03 g 1,4-Dihydro-6-methyl-4-(3-pyridyl)-pyridazin-3,5-dicarbonsäure-3-methyl-5-ethylester werden mit 2,0 g NBS und 150 g Azobisisobutyronitril in 50 ml Tetrachlorkohlenstoff 16 Stunden zum Rückfluß erhitzt. Nach Absaugen des Succinimids und Einengen der Lösung wird der Rückstand auf Kieselgel mit Chloroform + 3 % Methanol chromatographiert. ($R_f$=0.4)

Ausbeute: (41 % der Theorie).
Harz.

0173205

Beispiel 18

4-(2,1,3-Benzoxadiazol-4-yl)-5-oxo-1,4,5,7-tetrahydro-furo-[3,4-c]pyridazin-3-carbonsäuremethylester

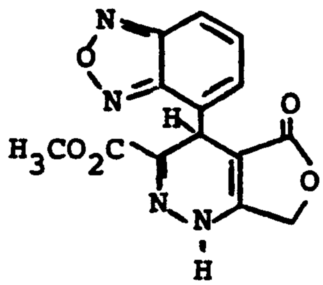

Analog Beispiel 17 wird 4-(2,1,3-Benzoxadiazol-4-yl)-1,4-dihydro-6-methyl-pyridazin-3,5-dicarbonsäuredimethylester umgesetzt.

Ausbeute: (36 % der Theorie).
Harz.

Beispiel 19

4-(4-Methoxyphenyl)-5-oxo-1,4,5,7-tetrahydro-furo [3,4-c]pyridazin-3-carbonsäuremethylester

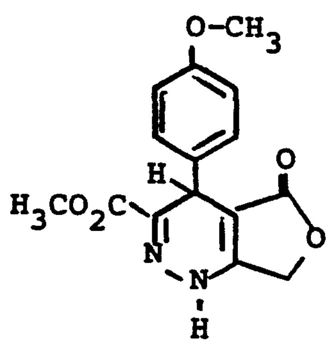

Analog Beispiel 17 wird 1,4-Dihydro-4-(4-methoxyphenyl)-6-methyl-pyridazin-3,5-dicarbonsäuredimethylester lactonisiert.

Ausbeute: (39 % der Theorie).
Harz.

Beispiel 20

4-(2-Chlorphenyl)-1-ethyl-5-oxo-1,4,5,7-tetrahydro-furo/3,4-c/-pyridazin-3-carbonsäureethylester

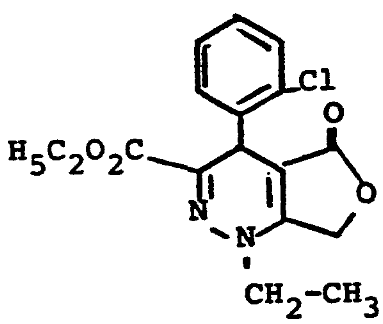

Analog Beispiel 17 wird 4-(2-Chlorphenyl)-1,4-dihydro-1-ethyl-6-methyl-pyridazin-3,5-dicarbonsäurediethylester umgesetzt.

Ausbeute: 18 % der Theorie.
Harz.

Patentansprüche:

1) 1,4-Dihydro-pyridazin-Derivate der allgemeinen Formel (I)

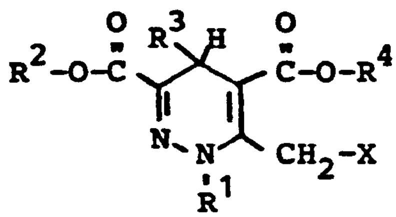

(I)

in welcher

$R^1$ für Wasserstoff, für einen geradkettigen oder verzweigten Alkylrest, der gegebenenfalls durch 1-2 Sauerstoffatome in der Kette unterbrochen ist,

oder für einen Aryl- oder Aralkylrest steht,

$R^2$ und $R^4$ gleich oder verschieden sind und jeweils für Wasserstoff, für einen geradkettigen, verzweigten oder cyclischen, gesättigten oder ungesättigten Kohlenwasserstoffrest stehen, der gegebenenfalls durch 1-2 Sauerstoff- und/oder Schwefelatome und/oder $SO_2$-Gruppen unterbrochen ist und/oder der substituiert sein kann durch Hydroxy, Halogen, Heteroaryl, eine gegebenenfalls durch Halogen, Cyan, Amino,

Trifluoromethyl, Alkyl, Alkylamino, Alkoxy oder Nitro substituierte Phenyl- oder Phenoxygruppe oder durch eine Aminogruppe, wobei diese Aminogruppe gegebenenfalls entweder Wasserstoff und einen Substituenten oder zwei gleiche oder verschiedene Substituenten aus den Gruppen Alkyl, Alkoxyalkyl, Aryl und Aralkyl trägt und wobei diese Substituenten gegebenenfalls mit dem Stickstoffatom einen 5- 7-gliedrigen Ring bilden, der als weitere Heteroatome ein Sauerstoff-, Schwefel- und/oder 1-2 Stickstoffatome enthalten kann und der mit Alkyl substituiert sein kann,

für Aryl, der gegebenenfalls 1-2 gleiche oder verschiedene Substituenten tragen kann, wobei als Substituenten geradkettiges oder verzweigtes Alkyl, Halogen, Nitro, Trifluormethyl oder Trifluormethoxy gelten,

oder für einen Heteroaromaten stehen,

$R^3$ für Aryl oder für Heteroaryl steht,

wobei sowohl der Aryl- als auch der Heteroarylrest gegebenenfalls 1-3 gleiche oder verschiedene Substituenten aus der Reihe Phenyl, Alkyl, Alkoxy, Halogen, Dioxyalkylen, Trifluormethyl, Trifluormethoxy, Cyan, Hydroxy, Azido, Nitro, Carboxy, Alkoxycarbonyl, Carbonamido, Sulfonamido, $SO_2$-$CF_3$, $SO_2$-Alkyl trägt.

X für Halogen steht

oder

X und $R^4$ gemeinsam für eine direkte Bindung stehen,

in Form von Isomeren, Isomerengemischen, Racematen und optischen Antipoden sowie deren physiologisch unbedenkliche Salze.

2) 1,4-Dihydro-pyridazin-derivate der allgemeinen Formel (I), gemäß Anspruch 1, in welcher

$R^1$ für Wasserstoff, für gerades oder verzweigtes Alkyl mit bis zu 8 C-Atomen, wobei der Alkylrest gegebenenfalls durch 1-2, bevorzugt 1 Sauerstoffatom in der Kette unterbrochen ist, oder für Aryl, Aralkyl mit jeweils 6-12 C-Atomen steht,

$R^2$ und $R^4$ gleich oder verschieden sind und jeweils für Wasserstoff, für einen geradkettigen, verzweigten oder cyclischen, gesättigten oder ungesättigten Kohlenwasserstoffrest mit bis zu 8 C-Atomen, der gegebenenfalls durch 1-2 Sauerstoff- und/oder Schwefelatome und/oder $SO_2$-Gruppen unterbrochen ist und/oder der substituiert sein kann durch Hydroxy, Fluor, Chlor, Brom, Heteroaryl bevorzugt Pyridyl,

Thienyl, Furyl, eine gegebenenfalls durch Fluor, Chlor, Brom, Cyan, Trifluormethyl, Alkyl mit 1-4 C-Atomen, Alkoxy mit 1-4 C-Atomen oder Nitro substituierte Phenyl- oder Phenoxygruppe oder durch eine Aminogruppe, wobei diese Aminogruppe gegebenenfalls entweder Wasserstoff und einen oder 2 gleiche oder verschiedene Substituenten aus den Gruppen Alkyl, Alkoxyalkyl (jeweils mit bis zu 4 C-Atomen), Aryl und Aralkyl (mit bis zu 12 C-Atomen) trägt und wobei diese Substituenten gegebenenfalls mit dem Stickstoffatom einen 5-6 gliedrigen Ring bilden, der als weiteres Atom ein Sauerstoff-, Schwefel- oder Stickstoffatom enthalten kann und gegebenenfalls mit Alkyl mit 1-4 C-Atomen substituiert sein kann,

für einen Phenylrest, der gegebenenfalls 1-2 gleiche oder verschiedene Substituenten tragen kann, wobei die Substituenten geradkettiges oder verzweigtes Alkyl mit bis zu 4 C-Atomen, Fluor, Chlor, Brom, Nitro, Trifluormethyl oder Trifluormethoxy sein können,

oder für Pyridyl, Thienyl oder Furyl stehen,

$R^3$ für Phenyl, Naphthyl, für Pyrryl, Furyl, Thienyl, Pyrazolyl, Imidazolyl, Oxazolyl, Isoxazolyl, Thiazolyl,

Pyridyl, Pyridazinyl, Pyrimidyl, Pyrazinyl, Chinolyl, Isochinolyl, Indolyl, Benzoxadiazolyl, Benzimidazolyl, Chinazolyl oder Chinoxalyl steht, wobei sowohl Aryl als auch Heteroaryl gegebenenfalls 1-2 gleiche oder verschiedene Substituenten aus der Reihe Phenyl, Alkyl, Alkoxy jeweils mit 1-4 C-Atomen, Fluor, Chlor, Brom, Dioxymethylen, Trifluormethyl, Trifluormethoxy, Cyan, Hydroxy, Azido, Nitro, Carboxy, Alkoxycarbonyl mit 2-4 C-Atomen, Carbonamido, Sulfonamido, $SO_2-CH_3$, $SO_2$-Alkyl mit 1-4 C-Atomen tragen,

X für Halogen, bevorzugt Chlor oder Brom, steht

oder

X und $R^4$ gemeinsam eine direkte Bindung darstellen.

3) 1,4-Dihydro-pyridazin-derivate der allgemeinen Formel (I) gemäß Anspruch 1, in welcher

$R^1$ für Wasserstoff, für Alkyl mit bis zu 5 C-Atomen, gegebenenfalls durch ein Sauerstoffatom in der Kette unterbrochen, oder für Phenyl oder Benzyl steht,

$R^2$ und $R^4$ gleich oder verschieden sind und jeweils für Wasserstoff,

für einen geradkettigen, verzweigten oder cyclischen, gesättigten oder ungesättigten Kohlenwasserstoffrest mit bis zu 6 C-Atomen, der gegebenenfalls durch bis zu 2 Sauerstoff- und/oder Schwefelatome und/oder $SO_2$-Gruppen unterbrochen ist und/oder der substituiert sein kann durch Hydroxy, ein oder mehrere Fluor, Pyridyl, Furyl, Thienyl, eine gegebenenfalls durch Fluor, Chlor, Brom, Nitro oder Trifluormethyl substituierte Phenyl- oder Phenoxygruppe oder durch eine Aminogruppe, wobei diese Aminogruppe gegebenenfalls entweder Wasserstoff und einen oder zwei gleiche oder verschiedene Substituenten aus den Gruppen Alkyl, Alkoxy mit jeweils bis zu 2 C-Atomen, Phenyl, Benzyl trägt,
oder wobei diese Substituenten mit dem Stickstoffatom die gegebenenfalls mit Methyl oder Ethyl substituierten Ringsysteme Pyrrolidin, Pyrazolidin, Piperidin, Piperazin, Morpholin, Thiomorpholin bildet,

für einen Phenylrest, der gegebenenfalls 1-2 gleiche oder verschiedene Substituenten tragen kann, wobei die Substituenten Alkyl mit 1-2 C-Atomen, Fluor, Chlor, Brom, Nitro, Trifluormethyl oder Trifluormethoxy sein können,

oder für Pyridyl, Furyl oder Thienyl stehen,

$R^3$ für Phenyl, gegebenenfalls substituiert durch ein oder zwei gleiche oder verschiedene Substituenten aus den Gruppen Alkyl oder Alkoxy, jeweils mit 1-2 C-Atomen, Fluor, Chlor, Brom, Trifluormethyl, Trifluormethoxy, Nitro, Azido und Cyano, oder für Benzoxadiazolyl, Pyridyl, Thienyl oder Furyl steht,

X für Chlor oder Brom steht,

oder

$R^4$ und X gemeinsam eine direkte Bindung darstellen.

4) Verfahren zur Herstellung der Verbindungen der allgemeinen Formel (Ia)

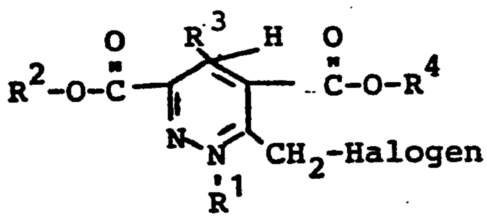

(Ia)

in welcher

$R^1$ für Wasserstoff, für einen geradkettigen oder verzweigten Alkylrest, der gegebenenfalls durch 1-2 Saurstoffatome in der Kette unterbrochen ist,

oder für einen Aryl- oder Aralkylrest steht,

$R^2$ und $R^4$ gleich oder verschieden sind und jeweils für Wasserstoff, für einen geradkettigen, verzweigten oder cyclischen, gesättigten oder ungesättigten Kohlenwasserstoffrest stehen, der gegebenenfalls durch 1-2 Sauerstoff- und/oder Schwefelatome und/oder $SO_2$-Gruppen unterbrochen ist und/oder der substituiert sein kann durch Hydroxy, Halogen, Heteroaryl, eine gegebenenfalls durch Halogen, Cyan, Amino, Trifluormethyl, Alkyl, Alkylamino, Alkoxy oder Nitro substituierte Phenyl- oder Phenoxygruppe oder durch eine Aminogruppe, wobei diese Aminogruppe gegebenenfalls entweder Wasserstoff und einen Substituenten oder zwei gleiche oder verschiedene Substituenten aus den Gruppen Alkyl, Alkoxyalkyl, Aryl und Aralkyl trägt und wobei diese Substituenten gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der mit Alkyl substituiert sein kann,

für Aryl, der gegebenenfalls 1-2 gleiche oder verschiedene Substituenten tragen kann, wobei als Substituenten geradkettiges oder verzweigtes Alkyl, Halogen, Nitro, Trifluormethoxy gelten,

oder für einen Heteroaromaten stehen,

$R^3$ für einen Arylrest oder für Heteroaryl steht,

wobei sowohl der Aryl- als auch der Heteroarylrest gegebenenfalls 1-3 gleiche oder verschiedene Substituenten aus der Reihe Phenyl, Alkyl, Alkoxy, Halogen, Dioxyalkylen, Trifluormethyl, Trifluormethoxy, Cyan, Hydroxy, Azido, Nitro, Carboxy, Alkoxycarbonyl, Carbonamido, Sulfonamido, $SO_2$-$CF_3$, $SO_2$-Alkyl trägt,

X für Halogen steht,

dadurch gekennzeichnet, daß man 1,4-Dihydropyridazine der allgemeinen Formel (II)

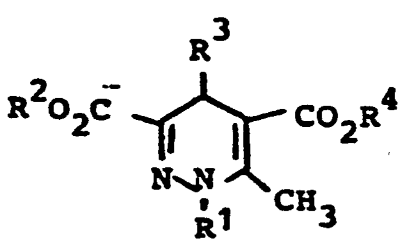

(II)

in welcher

$R^1$ - $R^4$ die oben angegebene Bedeutung haben

mit Halogenierungsmitteln in Gegenwart von inerten organischen Lösungsmitteln, gegebenenfalls in Anwesenheit von Radikalbildnern umsetzt.

5) Verfahren gemäß Anspruch 4 dadurch gekennzeichnet, daß man die Umsetzung mit Chlor, Brom, N-Chlorsuccinimid, N-Bromsuccinimid gegebenenfalls in Anwesenheit von Radikalbildnern durchführt.

6) Verfahren gemäß Anspruch 4 dadurch gekennzeichnet, daß man die Umsetzung in einem Temperaturbereich von 30 - 120°C durchführt.

7) Verfahren zur Herstellung der Verbindungen der allgemeinen Formel (I b)

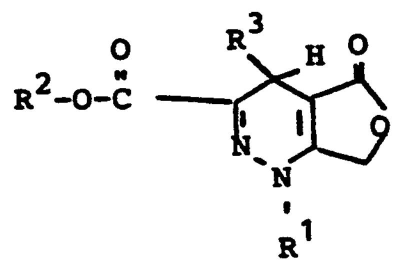

(Ib)

in welcher

$R^1$ für Wasserstoff, für einen geradkettigen oder verzweigten Alkylrest steht, der gegebenenfalls durch 1-2 Sauerstoffatome in der Kette unterbochen ist,

oder für einen Aryl- oder Aralkylrest steht,

$R^2$ für Wasserstoff, für einen geradkettigen, verzweigten oder cyclischen, gesättigten oder ungesättigten Kohlenwasserstoffrest steht, der gegebenenfalls durch 1-2 Sauerstoff- und/oder Schwefelatome

und/oder $SO_2$-Gruppen unterbrochen ist und/oder der substituiert sein kann durch Hydroxy, Halogen, Heteroaryl, eine gegebenenfalls durch Halogen, Cyan, Amino, Trifluormethyl, Alkyl, Alkylamino, Alkoxy oder Nitro substituierte Phenyl- oder Phenoxygruppe oder durch eine Aminogruppe, wobei diese Aminogruppe gegebenenfalls entweder Wasserstoff und einen Substituenten oder zwei gleiche oder verschiedene Substituenten aus den Gruppen Alkyl, Alkoxyalkyl, Aryl und Aralkyl trägt und wobei diese Substituenten gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der mit Alkyl substituiert sein kann,

für Aryl steht, der gegebenenfalls 1-2 gleiche oder verschiedene Substituenten tragen kann, wobei als Substituenten geradkettiges oder verzweigtes Alkyl, Halogen, Nitro, Trifluormethyl oder Trifluormethoxy gelten,

oder für einen Heteroaromaten steht,

$R^3$ für Aryl oder für Heteroaryl steht, wobei sowohl der Aryl- als auch der Heteroarylrest gegebenenfalls 1-3 gleiche oder verschiedene Substituenten aus der Reihe Phenyl, Alkyl, Alkoxy, Halogen, Dioxyalkylen, Trifluormethyl, Trifluormethoxy, Cyan, Hydroxy, Azido, Nitro, Carboxy, Alkoxycarbonyl, Carbonamido, Sulfonamido, $SO_2$-$CF_3$, $SO_2$-Alkyl trägt,

dadurch gekennzeichnet, daß man 1,4-Dihydro-pyridazine der Formel (Ia) gemäß Anspruch 4 mit oder ohne Lösungsmittel pyrolisiert.

8) Verfahren gemäß Anspruch 7 dadurch gekennzeichnet, daß man die Cyclisierung in einem Temperaturbereich von 20 - 300°C durchführt.

9) Verbindungen gemäß den Ansprüchen 1-3 zur Bekämpfung von Krankheiten.

10) Verbindungen gemäß den Ansprüchen 1-3 zur Bekämpfung von Kreislauferkrankungen.

11) Arzneimittel enthaltend als Wirkstoff mindestens eine Verbindung der allgemeinen Formel (I) gemäß den Ansprüchen 1-3.

12) Verfahren zur Herstellung eines Arzneimittels gemäß Anspruch 11 dadurch gekennzeichnet, daß man die Wirkstoffe mit den üblichen Hilfs- und/oder Trägerstoffen mischt.

13) Verwendung von Stoffen gemäß Ansprüchen 1-3 zur Bekämpfung von Erkrankungen.

14) Verwendung von Stoffen, gemäß den Ansprüchen 1-3 zur Bekämpfung von Kreislauferkrankungen.